# EUROPEAN PATENT APPLICATION

(11) **EP 1 698 276 A1**
(43) Date of publication of application: **06.09.2006**
(21) Application number: 06251166.2
(22) Date of filing: 03.03.2006
(51) Int. Cl.: A61B 5/0215

(54) **Endocardial pressure differential sensing systems**

(30) Priority: 03.03.2005 US 72942
(71) Applicant: PACESETTER, INC., Sylmar, CA 91392-9221 (US)
(72) Inventor: Fayram, Timothy A., Gilroy, CA 95020 (US); Turcott, Robert G., Mountain View CA 94040 (US)
(74) Representative: Rees, David Christopher

(57) **Abstract**

An implanted system that monitors a cardiac condition. Pressure is sensed in a left chamber of the heart and a right chamber of the heart. A pressure differential is determined between the sensed pressures and the cardiac condition is monitored based on the pressure differential. By determining pressure differentials, as opposed to absolute pressures, calibrations/adjustments for changes in weather, altitude or similar pressure affecting factors are not necessary, since the pressure in the left and right chambers should both be equally affected by such changes. Accordingly, an external (i.e., non-implanted) pressure sensor is not needed for measuring ambient pressure.

## Description

The present invention relates generally to implantable devices that are capable of measuring endocardial pressure.

Heart failure is a condition in which a patient's heart works less efficiently than it should, resulting in the heart failing to supply the body sufficiently with the oxygen rich blood it requires, either at exercise or at rest. Congestive heart failure (CHF) is heart failure accompanied by a build-up of fluid pressure in the pulmonary blood vessels that drain the lungs. Transudation of fluid from the pulmonary veins into the pulmonary interstitial spaces, and eventually into the alveolar air spaces, is called pulmonary edema, and can cause shortness of breath, hypoxernia, acidosis, respiratory arrest, and even death.

Chronic diseases such as CHF require close medical management to reduce morbidity and mortality. Because the disease status evolves with time, frequent physician follow-up examinations are typically necessary. At follow-up, the physician may make adjustments to the drug regimen in order to optimize therapy. This conventional approach of periodic follow-up is unsatisfactory for some diseases, such as CHF, in which acute, life-threatening exacerbations can develop between physician follow-up examinations. It is well know among clinicians that if a developing exacerbation is recognized early, it can be more easily and inexpensively terminated, typically with a modest increase in oral diuretic. However, if it develops beyond the initial phase, an acute heart failure exacerbation becomes difficult to control and terminate. Hospitalization in an intensive care unit is often required. It is during an acute exacerbation of heart failure that many patients succumb to the disease.

It is often difficult for patients to subjectively recognize a developing exacerbation, despite the presence of numerous physical signs that would allow a physician to readily detect it. Furthermore, since exacerbations typically develop over hours to days, even frequently scheduled routine follow-up with a physician cannot effectively detect most developing exacerbations. It is therefore desirable to have a system that allows the routine, frequent monitoring of patients so that an exacerbation can be recognized early in its course. With the patient and/or physician thus notified by the monitoring system of the need for medical intervention, a developing exacerbation can more easily and inexpensively be terminated early in its course.

Accordingly, it would be advantageous to provide implantable cardiac devices that can obtain complex and informative trends about a patient's heart failure progression. More generally, it is desirable to provide implantable cardiac devices that can obtain disease progression information.

It has been suggested that pressure sensors can be chronically implanted within various chambers of the heart for the purpose of monitoring diseases such as heart failure. Expected systolic/diastolic pressures in left atrium are in the range of about 10 to 20 / 5 mm Hg, and is indicative of left ventricular filling pressure. In clinical settings left atrial pressure is estimated by the pulmonary capillary wedge pressure, which is obtained by occluding a small branch of the pulmonary artery using a pressure-tipped catheter. The pressure of the static fluid column distal to the catheter reflects the left atrial pressure. Expected systolic/diastolic pressure in the right atrium is in the range of about 5 to 8 / 0 to 3 mm Hg, and is indicative of both central venous pressure, which reflects the total blood volume, and right ventricular filling pressure (steady state venous pressure and with a linear correlation factor that could be added to right atrial pressure, as an indication of right ventricular and pulmonary artery pressures).

However, measurements made by implanted pressure sensors may be affected by changes in ambient pressure that result, e.g., from changes in weather and/or altitude. For a more specific example, when a person having an implanted pressure sensor drives up a mountain, or ascends in an airplane, measurements from the implanted pressure sensor may indicate an decrease in pressure. Such confounding factors may compromise the ability of an implanted system to detect an exacerbation, since the measured change in pressure is not due to physiologic changes. One way to overcome this problem is for the person to carry an external device that monitors ambient pressure, which can be used to calibrate/adjust the endocardial pressure measurements. More specifically, the ambient pressure measurements from the external device and the endocardial pressure measurements from the implanted device can both be telemetered to a further device that uses the ambient measurements to appropriately calibrate/adjust the endocardial pressure measurements. Alternatively, the external device can wirelessly transmit the ambient pressure measurements to the implanted system, which can then appropriately calibrate/adjust the endocardial pressure measurements. However, a disadvantage of this approach is that the patient needs to carry an external device, which is inconvenient, and may be forgotten or lost. Accordingly, it would be advantageous if methods and systems for monitoring endocardial pressure could account for changes in ambient pressure (e.g., due to weather and/or altitude changes) without requiring that a patient carry an external device.

According to the invention, there is provided an implantable system for monitoring a cardiac condition, characterised by: means for determining a pressure differential between pressure sensed in a left chamber and pressure sensed in a right chamber of the heart; and means for monitoring a cardiac condition based on the pressure differential. Preferably, the system comprises a first pressure sensor to be implanted in the left atrium or ventricle to sense pressure in a left chamber of the heart; and a second pressure sensor to be implanted in the right atrium or ventricle to sense pressure in a right chamber of the heart.

In one variant, the means for monitoring recognizes an increase in the pressure differential as a worsening of left-sided heart failure, and preferably, the means for monitoring detects a gradual increase in the pressure differential when the pressure differential increases by at least a specified threshold amount over a specified threshold time period or more.

However, possibly, the means for monitoring recognizes a rapid increase in the pressure differential, while pressure sensed in the right chamber remains relatively constant, as pulmonary edema, or the means for monitoring recognizes a rapid increase in the pressure differential, while pressure sensed in the left chamber remains relatively constant, as pulmonary hypertension. In either case, preferably, the means for monitoring detects a rapid increase in the pressure differential when the pressure differential increases by at least a specified threshold amount over a specified threshold time period or less.

In another variant, the means for monitoring recognizes a gradual decrease in the pressure differential as a worsening of right-sided heart failure, and preferably, the means for monitoring detects a gradual decrease in the pressure differential when the pressure differential decreases by at least a specified threshold amount over a specified threshold time period or more.

The cardiac condition may be endocardial flow and so preferably, the means for monitoring monitors endocardial flow.

In a preferred form, the invention provides an implantable system for monitoring a cardiac condition, characterised by: a first pressure sensor to be implanted in the left atrium or ventricle to sense pressure in a left chamber of the heart; a second pressure sensor to be implanted in the right atrium or ventricle to sense pressure in a right chamber of the heart; and a microcontroller to determine a pressure differential between pressure sensed by the first pressure sensor and pressure sensed by the second pressure sensor and to monitor a cardiac condition based on the pressure differential.

The system of the present invention permits a method for monitoring a cardiac condition, comprising (a) sensing pressure in a left chamber of the heart and sensing pressure in a right chamber of the heart; (b) determining a pressure differential between the sensed pressure in the left chamber and the sensed pressure in the right chamber; and (c) monitoring a cardiac condition based on the pressure differential. Step (a) may comprise: sensing pressure in the left atrium and sensing pressure in the right atrium; or sensing pressure in the left atrium and sensing pressure in the right ventricle; or sensing pressure in the left ventricle and sensing pressure in the right ventricle; or sensing pressure in the left ventricle and sensing pressure in the right atrium; or sensing pressure in the left atrium and sensing pressure in the right ventricle and the right atrium.

When the cardiac condition is left-sided heart failure, step (c) may include recognizing an increase in the pressure differential as a worsening of left-sided heart failure and preferably includes recognizing a gradual increase in the pressure differential as a gradual worsening of left-sided heart failure. Also, step (c) may include detecting a gradual increase in the pressure differential when the pressure differential increases by at least a specified threshold amount over a specified threshold time period or more. Alternatively step (c) may include detecting a rapid increase in the pressure differential when the pressure differential increases by at least a specified threshold amount over a specified threshold time period or less.

When the cardiac condition is pulmonary edema, step (c) may include recognizing a rapid increase in the pressure differential, while the sensed pressure in the right chamber remains relatively constant, as pulmonary edema, and preferably, step (c) includes detecting a rapid increase in the pressure differential when the pressure differential increases by at least a specified threshold amount over a specified threshold time period or less.

When the cardiac condition is pulmonary hypertension, step (c) include recognizing a rapid increase in the pressure differential, while the sensed pressure in the left chamber remains relatively constant, as pulmonary hypertension, and preferably, step (c) includes detecting a rapid increase in the pressure differential when the pressure differential increases by at least a specified threshold amount over a specified threshold time period or less.

When the cardiac condition is right-sided heart failure, step (c) may include recognizing a decrease in the pressure differential as a worsening of right-sided heart failure, and preferably, step (c) includes detecting a gradual decrease in the pressure differential when the pressure differential decreases by at least a specified threshold amount over a specified threshold time period or more.

Thus, embodiments of the present invention are directed to implantable systems for monitoring a cardiac condition. In accordance with embodiments of the present invention, pressure is sensed in a left chamber of the heart and pressure is sensed in a right chamber of the heart, so that a pressure differential can be determined (between the sensed pressure in the left chamber and the sensed pressure in the right chamber). A cardiac condition is monitored based on this pressure differential. By determining pressure differentials, as opposed to absolute pressures, calibrations/adjustments for changes in weather, altitude or similar pressure affecting factors are not necessary, since the pressure in the left and right chambers should both be equally affected by such changes. Accordingly, with embodiments of the present invention, an external (i.e., non-implanted) pressure sensor is not needed for measuring ambient pressure.

In one embodiment, pressure is sensed in the left and right chambers of the heart by sensing pressure in the left atrium and sensing pressure in the right atrium. In another embodiment, pressure is sensed in the left and right chambers of the heart by sensing pressure in the left atrium and sensing pressure in the right ventricle. In still another embodiment, this is accomplished by sensing pressure in the left ventricle and sensing pressure in the right ventricle. In a further embodiment this is accomplished by sensing pressure in the left ventricle and sensing pressure in the right atrium. In still a further embodiment this is accomplished by sensing pressure in the left atrium and sensing pressure in the right ventricle and the right atrium.

In one embodiment the cardiac condition that is being monitored is left-sided heart failure. In such an embodiment, an increase in the pressure differential may be recognized as a worsening of left-sided heart failure. More specifically, a gradual increase in the pressure differential may be recognized as a gradual worsening of left-sided heart failure, and a rapid increase in the pressure differential may be recognized as being indicative of a sudden onset of severe mitral regurgitation and/or flash pulmonary edema. Detecting a gradual increase in the pressure differential may occur when the pressure differential increases by at least a specified threshold amount over a specified threshold time period (e.g., of ten days) or more. Detecting a rapid increase in the pressure differential may occur when the pressure differential increases by at least a specified threshold amount over a specified threshold time period (e.g., of one hour) or less.

In another embodiment the cardiac condition that is being monitored is pulmonary edema. In such an embodiment, a rapid increase in the pressure differential, while the sensed pressure in the right chamber remains relatively constant, may be recognized as pulmonary edema. Detecting a rapid increase in the pressure differential may occur when the pressure differential increases by at least a specified threshold amount over a specified threshold time period (e.g., of two days) or less.

In a further embodiment, the cardiac condition that is being monitored is pulmonary hypertension. In such an embodiment, a rapid increase in the pressure differential, while the sensed pressure in the left chamber remains relatively constant, may be recognized as pulmonary hypertension. Detecting a rapid increase in the pressure differential may occur when the pressure differential increases by at least a specified threshold amount over a specified threshold time period (e.g., of two days) or less.

In still another embodiment, the cardiac condition that is being monitored is right-sided heart failure. In such an embodiment, a gradual decrease in the pressure differential may be recognized as a gradual worsening of right-sided heart failure. Detecting a gradual decrease in the pressure differential may occur when the pressure differential decreases by at least a specified threshold amount over a specified threshold time period (e.g., of ten days) or more.

In still a further embodiment, the cardiac condition that is being monitored is endocardial flow.

The invention may be carried into practice in various ways and some embodiments will now be described by way of example with reference to the accompanying drawings, in which:
FIG. 1A is a simplified diagram illustrating an exemplary implantable device in electrical communication with a patient's heart by means of multiple leads suitable for delivering multi-chamber stimulation and pacing therapy;
FIG. 1B is useful for describing how pressure sensors can be implanted within a patient's heart and connected by leads to the implantable device of FIG. 1A;
FIG. 2 is a functional block diagram of the exemplary implantable device, which can provide cardioversion, defibrillation, and pacing stimulation in four chambers of a heart, and can measure endocardial pressure, in accordance with embodiments of the present invention; and
FIG. 3 is a high level flow diagram that is useful for describing additional details of embodiments of the present invention.

It is believed that chronic monitoring of the pressures within the chambers of the heart will be important in future cardiac pulse generator applications. For example, left sided heart failure can be monitored by initially detecting an increased systolic and diastolic pressures in the left ventricle and left atrium. As the disease progresses into the right side of the heart, increased back pressure into the major venous system can be detected as well.

Existing non-implanted acute and invasive pressure measurement systems have excellent accuracy and precision. However, the specifications of a chronic pressure measurement system may not need to meet the performance specifications of the existing acute systems. Rather, the inventors of the present invention believe that differential measurements that allow the implanted system to measure general trends with time may suffice, and that calibration for drift in such a chronic system may not be necessary since the physician can, if desired, make a more precise diagnosis with at acute system at a regular follow up.

Before describing embodiments of the invention in additional detail, it is helpful to first describe an example environment in which embodiments of the invention may be implemented.

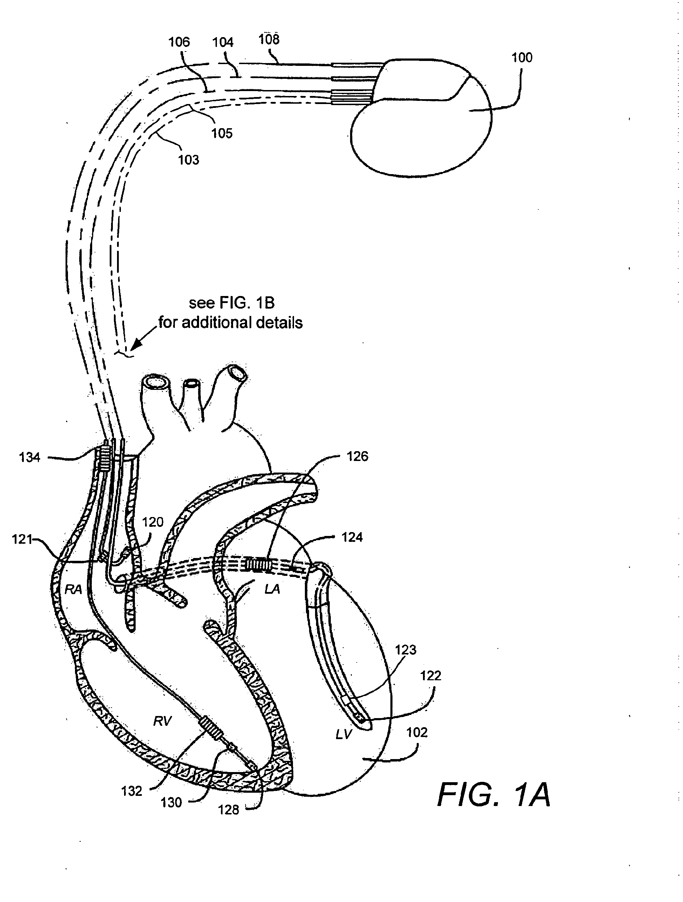

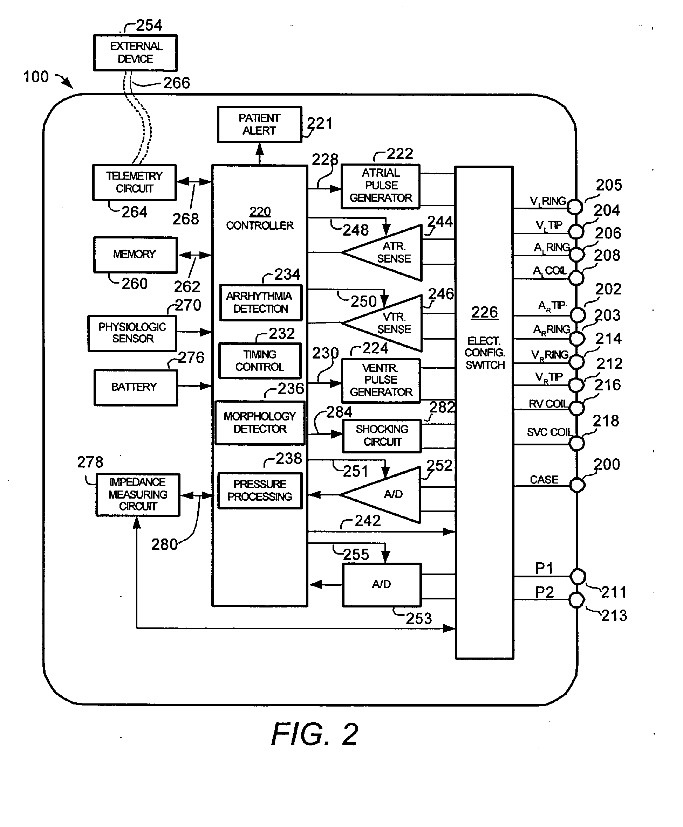

Embodiments of the present invention are particularly useful in the environment of an implantable cardiac device that may monitor electrical activity of a heart and deliver appropriate electrical therapy, including for example, pacing pulses, cardioverting and defibrillator pulses, and/or drug therapy, as required. Implantable cardiac devices include, for example, pacemakers, cardioverters, defibrillators, implantable cardioverter defibrillators, and the like. The term "implantable cardiac device" or simply "ICD" is used herein to refer to any implantable cardiac device, even those that don't deliver electrical stimulation (e.g., the implantable device may simply be a monitor that records data). FIGS. 1A and 2 illustrate such an environment in which embodiments of the present invention can be used.

Referring first to FIG. 1A, an exemplary implantable device 100 is shown as being in electrical communication with a patient's heart 102 by way of three leads 104, 106, and 108, suitable for delivering multi-chamber stimulation and shock therapy. To sense atrial cardiac signals and to provide right atrial chamber stimulation therapy, implantable device 100 is coupled to an implantable right atrial lead 104 having at least an atrial tip electrode 120, which typically is implanted in the patient's right atrial appendage or septum. FIG. 1A shows the right atrial lead 104 also as having an optional atrial ring electrode 121.

To sense left atrial and ventricular cardiac signals and to provide left chamber pacing therapy, implantable device 100 is coupled to a coronary sinus lead 106 designed for placement in the coronary sinus region via the coronary sinus for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, an exemplary coronary sinus lead 106 is designed to receive atrial and ventricular cardiac signals and to deliver left ventricular pacing therapy using at least a left ventricular tip electrode 122, left ventricular ring electrode 123, left atrial pacing therapy using at least a left atrial ring electrode 124, and shocking therapy using at least a left atrial coil electrode 126 (or other electrode capable of delivering a shock). For a more complete description of a coronary sinus lead, the reader is directed to U.S. Patent No. 5,466,254.

Implantable device 100 is also shown in electrical communication with the patient's heart 102 by way of an implantable right ventricular lead 108 having, in this implementation, a right ventricular (RV) tip electrode 128, a right ventricular ring electrode 130, a right ventricular coil electrode 132 (or other electrode capable of delivering a shock), and superior vena cava (SVC) coil electrode 134 (or other electrode capable of delivering a shock). Typically, the right ventricular lead 108 is transvenously inserted into the heart 102 to place the right ventricular tip electrode 128 in the right ventricular apex so that the RV coil electrode 132 will be positioned in the right ventricle and the SVC coil electrode 134 will be positioned in the superior vena cava. Accordingly, the right ventricular lead 108 is capable of sensing or receiving cardiac signals, and delivering stimulation in the form of pacing and shock therapy to the right ventricle.

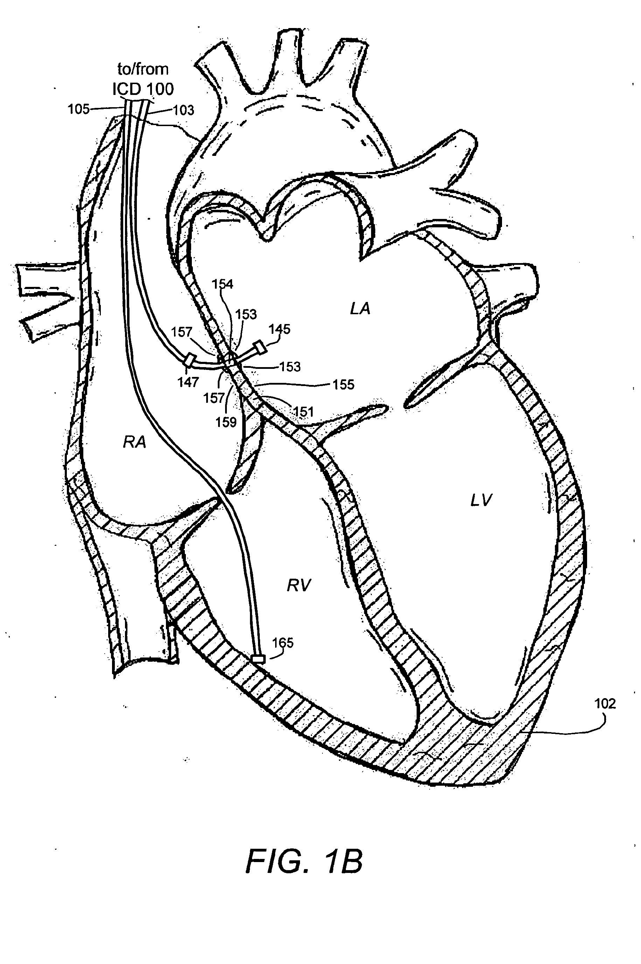

Also shown in FIG. 1A are leads 103 and 105 to which are attached pressure sensors, not shown in FIG. 1A, but shown in FIG. 1B (which is a cutaway view of the heart 102 from a different angle than shown in FIG. 1B). Referring now to FIG. 1B, the lead 103 includes two pressure sensor 145 and 147, one of which is located in the left atrium (LA), and one of which is located in the right atrium (RA). In this embodiment, the distal tip of the lead 103 contains the left atrial pressure sensor 145. A few millimeters to a few centimeters behind the distal tip of the lead 103 is the right atrial pressure sensor 147 located on the annulus of the lead 103. As will be described below, the implantable device 100 includes circuitry that processes signals from the sensors 145 and 147 to determine pressure differentials, in accordance with embodiments of the present invention.

To pass the lead 103 through to the left atrium (LA), the atrial septal wall 151 may be pierced using, for example, a piercing guide wire tool (not shown), or using a lead 103 that includes on its distal end a relatively sharp and hard tip (not shown), or using a lead that includes a deployable and retractable piercing mechanism. The piercing apparatus is manipulated to create an access tunnel 154 in the septum 151. The access tunnel 154 may be made in the region of the fossa ovalis since this may be the thinnest portion of the atrial septum 151.

The distal portion of the lead 103 is then maneuvered through the atrial septum 151 (e.g., using the stylet) so that all or a portion of the pressure sensor 145 at the distal end of the lead 103 protrudes into the left atrium. In this way, the sensor 145 may be used to accurately measure pressure in the left atrium.

The lead 103 also may include another pressure sensor 147 positioned proximally on the lead from the sensor 145. The sensor 147 may thus be used to measure pressure in the right atrium.

The lead 103 can include an attachment structure that serves to attach the lead 103 to the septum 151. The attachment structure may take many forms including, without limitation, one or more tines, flexible membranes, inflatable membranes, circumferential tines and/or J-leads. FIG. 1B represents the attachment structure in a generalized manner.

In the embodiment of FIG. 1B, the attachment structure includes a first attachment structure 153 and a second attachment structure 157 implanted on opposite sides of the septum 151. In other applications a single attachment structure may be implanted on one of the sides of the septum 151.

In accordance with an embodiment, the first attachment structure 153 is attached to the distal portion of the lead 103. After the first attachment structure 153 is pushed through an access tunnel 154 pierced through the septum 151, it expands outwardly from the lead 103 such that it tends to prevent the distal end of the lead 103 from being pulled back through the access tunnel 154. The first attachment structure 153 is then positioned against a septal wall 155 in the left atrium.

The second attachment structure 157 extends outwardly from the lead 103 to help prevent the lead 103 from sliding further down into the left atrium. As FIG 1B illustrates, the second attachment structure 157 is positioned against a septal wall 159 in the right atrium.

In some embodiments the attachment structures 153 and 157 are positioned a predefined distance apart on the lead 103. For example, the lead may be constructed so that the spacing between the attachment structures 153 and 157 is approximately equal to the thickness of the septum 151 in the area of the access tunnel 154. In some embodiments the attachment structures are retractable to facilitate subsequent lead extraction.

In some embodiments, one or more of the attachment structures 153 and 157 are attached to the lead 103 in a manner that enables the position of the attachment structure to be adjusted. For example, one or both of the attachment structures 153 and 157 may be slideably mounted to the lead 103 so that they may be moved toward one another to firmly place each attachment structure against the septum 151. Such movement of the attachment structures 153 and 157 may be accomplished, for example, by a manual operation (e.g., via a tensile member such as a stylet or a sheath) or automatically through the use of a biasing member (e.g., a spring).

The attachment structures are preferably configured so that they have a relatively low profile against the septal wall 151. In this way, problems associated with protruding objects in the side of the heart may be avoided. For example, it is possible that blood clots may form on an object that protrudes from a wall of the heart. If these blood clots break loose in the left side of the heart the blood clots may travel to other areas of the body such as the brain and cause a blockage in a blood vessel (i.e., an embolism). By configuring the attachment(s) to have a low profile, a biological layer of endothelial cells ("the intima") may quickly build up over the attachment structure. As a result, the likelihood of blood clots breaking loose may be significantly reduced.

The buildup of the intima also may assist in firmly attaching the attachment structure(s) 153 and/or 157 to the septal wall 151. As a result, the lead 103 may be attached to the heart in a sufficiently stable manner so as to prevent injury to the heart and provide accurate pressure measurements.

The lead 103 also may include an electrode (not shown) that may be used to apply stimulation signals to the septum 151. For example, a circumferential electrode such as a ring electrode may be located between the first and second attachment structures 153 and 157.

Various control apparatus may be attached to the proximal end of the lead 103. For example, mechanisms may be provided for moving stylets or guide wires, movable sheaths or other components (not shown) in the lead 103 or for controlling the flow of fluid through lumens in the lead 103. In some applications, the control apparatus may be removed from the lead 103 when the device 100 (not shown in FIG. 1B) attached to proximal end of the lead is implanted in the patient. In other embodiments, one or more of the pressure sensors can be attached to the same leads that are used for measuring electrical activity and/or delivering electrical stimulations to the various chambers of the heart. For example, the left atrial pressure sensor 145 can be connected to the portion of the coronary sinus lead 106 (shown in FIG. 1A) that is located in the left atrium. For another example, the right atrial pressure sensor 147 can be connected to the right atrial lead 104.

The inventors of the present invention believe that changes in right atrial pressure reflect similar changes in right ventricular pressure, and vise versa. Similarly, it is believed that changes in the left atrial pressure will result in similar changes in the left ventricle, and vice versa. In addition, right ventricular systolic pressure, in the absence of pulmonic stenosis, is equal to pulmonary artery systolic pressure.
Finally, pulmonary artery diastolic pressure can be estimated from the RV pressure waveform. Accordingly, a pressure sensor 165 can be placed in the right ventricle (RV) rather than, or in addition to, being placed in the right atrium. As shown in FIG. 1B, such a right ventricular pressure sensor 165 can be attached to its own dedicated lead 105, which can be anchored to a right ventricle wall using an anchor mechanism similar to those described above. Alternatively, the right pressure sensor 165 can be attached to the right ventricular lead 108, or to any other lead that extends through the right ventricle. Similarly, a pressure sensor can be place in the left ventricle (LV) rather than, or in addition to, being placed in the left ventricle.

The pressure sensors can be analog devices that produce analog signals, or digital devices that produce digital signals. An example of an ultra small digital pressure sensor die include the SM5201 from Silicon Microstructures Incorporated (SMI) in Milpitas, CA.. An example of an ultra small analog pressure sensor die include the SM5112 from Silicon Microstructures Incorporated (SMI) in Milpitas, CA.. It is also possible that a hollow lumen catheter can be inserted within a heart chamber, with the hollow lumen catheter being in communication with a pressure transducer located within the housing of the implantable device 100. In such an embodiment, it will still be stated that the pressure sensor is located within a chamber of the heart since the hollow lumen can be considered part of the sensor.

Each lead (e.g., 103 and 105) can include a lead body that may house one or more electrical conductors, fluid-carrying lumens and/or other components (not shown). For example, a lead to which two pressure sensors are attached may include four conductors, one for providing an excitation voltage required to power a sensor, one for ground, one to carry the analog or digital pressure signal produced by one of the sensors, and another to carry the analog or digital pressure signal produced by the other sensor. Alternatively, if two pressure signals are interleaved on the same conductor using time-division multiplexing, then three conductors can be used with two pressure sensors attached to the same lead. It is also possible that each sensor (e.g., 145 and 147) can be connected to its own dedicated lead.

Each lead could be connected to a device header with, e.g., an IS-4 connector assembly. The implanted device could then process the independent left atrial and right atrial and/or right ventricle pressure signals and make various calculations based from the signals provided.

While specific techniques for implanting pressure sensors have been described above, this was merely for completeness. Embodiments of the present invention can be used with all techniques for placement of pressure sensors.

Through the use of the above described leads and sensors, and, in some cases, other leads and sensors implanted in the patient, the implantable cardiac device 100 can be used to provide a variety of cardiac pressure differential measurements in real time. These cardiac pressure differential measurements (also referred to as endocardial pressure differentials, or simply pressure differentials) can provide valuable information for monitoring and/or diagnosing a variety of cardiac problems, as will be described below. However, before going into more details about how embodiments of the present invention utilize pressure differential measurements, additional details of the exemplary implantable device 100 will be discussed in conjunction with FIG. 2.

FIG. 2 shows a simplified block diagram depicting various components of the exemplary implantable device 100. The implantable device 100, as shown, can be capable of treating both fast and slow arrhythmias with stimulation therapy, including cardioversion, defibrillation, and pacing stimulation. While a particular multi-chamber device is shown, it is to be appreciated and understood that this is done for illustration purposes only. Thus, the techniques and methods described below can be implemented in connection with any suitably configured or configurable implantable device. Accordingly, one of skill in the art could readily duplicate, eliminate, or disable the appropriate circuitry in any desired combination.

A housing 200 for the implantable device 100 is often referred to as the "can", "case" or "case electrode", and may be programmably selected to act as the return electrode for all "unipolar" modes. The housing 200 may further be used as a return electrode alone or in combination with one or more of the coil electrodes 126, 132 and 134 for shocking purposes. The housing 200 further includes a connector (not shown) having a plurality of terminals 202, 204, 206, 208, 212, 214, 216, and 218 (shown schematically and, for convenience, the names of the electrodes to which they are connected are shown next to the terminals).

To achieve right atrial sensing and pacing, the connector includes at least a right atrial tip terminal (AR TIP) 202 adapted for connection to the atrial tip electrode 120. A right atrial ring terminal (AR RING) 203 may also be included adapted for connection to the atrial ring electrode 121. To achieve left chamber sensing, pacing, and shocking, the connector includes at least a left ventricular tip terminal (VL TIP) 204, left ventricular ring terminal (VL RING) 205, a left atrial ring terminal (AL RING) 206, and a left atrial shocking terminal (AL COIL) 208, which are adapted for connection to the left ventricular tip electrode 122, the left atrial ring electrode 124, and the left atrial coil electrode 126, respectively.

To support right chamber sensing, pacing, and shocking, the connector further includes a right ventricular tip terminal (VR TIP) 212, a right ventricular ring terminal (VR RING) 214, a right ventricular shocking terminal (RV COIL) 216, and a superior vena cava shocking terminal (SVC COIL) 218, which are adapted for connection to the right ventricular tip electrode 128, right ventricular ring electrode 130, the RV coil electrode 132, and the SVC coil electrode 134, respectively.

At the core of the implantable device 100 is a programmable microcontroller 220 that controls the various modes of stimulation therapy. As is well known in the art, microcontroller 220 typically includes a microprocessor, or equivalent control circuitry, designed specifically for controlling the delivery of stimulation therapy, and may further include RAM or ROM memory, logic and timing circuitry, state machine circuitry, and/or I/O circuitry. Typically, microcontroller 220 includes the ability to process and/or monitor input signals (data or information) as controlled by a program code stored in a designated block of memory (e.g., memory 260). The type of microcontroller is not critical to the described implementations. Rather, any suitable microcontroller 220 may be used that carries out the functions described herein. The use of microprocessor-based control circuits for performing timing and data analysis functions are well known in the art.

Representative types of control circuitry that may be used in connection with the described embodiments can include the microprocessor-based control system of U.S. Patent No. 4,940,052 (Mann et al.), the state-machine of U.S. Patent Nos. 4,712,555 (Thornander et al.) and 4,944,298 (Sholder). For a more detailed description of the various timing intervals used within the implantable device and their inter-relationship, see U.S. Patent 4,788,980 (Mann et al.).

FIG. 2 also shows an atrial pulse generator 222 and a ventricular pulse generator 224 that generate pacing stimulation pulses for delivery by the right atrial lead 104, the coronary sinus lead 106, and/or the right ventricular lead 108 via an electrode configuration switch 226. It is understood that in order to provide stimulation therapy in each of the four chambers of the heart, the atrial and ventricular pulse generators, 222 and 224, may include dedicated, independent pulse generators, multiplexed pulse generators, or shared pulse generators. The pulse generators 222 and 224 are controlled by the microcontroller 220 via appropriate control signals 228 and 230, respectively, to trigger or inhibit the stimulation pulses.

Microcontroller 220 can also include timing control circuitry 232 to control the timing of the stimulation pulses (e.g., pacing rate, atrio-ventricular (AV) delay, atrial interconduction (A-A) delay, or ventricular interconduction (V-V) delay, etc.) as well as to keep track of the timing of refractory periods, blanking intervals, noise detection windows, evoked response windows, alert intervals, marker channel timing, etc., which is well known in the art.

Microcontroller 220 further includes an arrhythmia detector 234, a morphology detector 236, and optionally an orthostatic compensator and a minute ventilation (MV) response module, the latter two are not shown in FIG. 2. These components can be utilized by the implantable device 100 for determining desirable times to administer various therapies, including those to reduce the effects of orthostatic hypotension. The aforementioned components may be implemented in hardware as part of the microcontroller 220, or as software/firmware instructions programmed into the device and executed on the microcontroller 220 during certain modes of operation.

The electronic configuration switch 226 includes a plurality of switches for connecting the desired electrodes to the appropriate I/O circuits, thereby providing complete electrode programmability. Accordingly, switch 226, in response to a control signal 242 from the microcontroller 220, determines the polarity of the stimulation pulses (e.g., unipolar, bipolar, combipolar, etc.) by selectively closing the appropriate combination of switches (not shown) as is known in the art.

Atrial sensing circuits 244 and ventricular sensing circuits 246 may also be selectively coupled to the right atrial lead 104, coronary sinus lead 106, and the right ventricular lead 108, through the switch 226 for detecting the presence of cardiac activity in each of the four chambers of the heart. Accordingly, the atrial (ATR. SENSE) and ventricular (VTR. SENSE) sensing circuits, 244 and 246, may include dedicated sense amplifiers, multiplexed amplifiers, or shared amplifiers. Switch 226 determines the "sensing polarity" of the cardiac signal by selectively closing the appropriate switches, as is also known in the art. In this way, the clinician may program the sensing polarity independent of the stimulation polarity. The sensing circuits (e.g., 244 and 246) are optionally capable of obtaining information indicative of tissue capture.

Each sensing circuit 244 and 246 preferably employs one or more low power, precision amplifiers with programmable gain and/or automatic gain control, bandpass filtering, and a threshold detection circuit, as known in the art, to selectively sense the cardiac signal of interest. The automatic gain control enables the device 100 to deal effectively with the difficult problem of sensing the low amplitude signal characteristics of atrial or ventricular fibrillation.

The outputs of the atrial and ventricular sensing circuits 244 and 246 are connected to the microcontroller 220, which, in turn, is able to trigger or inhibit the atrial and ventricular pulse generators 222 and 224, respectively, in a demand fashion in response to the absence or presence of cardiac activity in the appropriate chambers of the heart. Furthermore, the microcontroller 220 is also capable of analyzing information output from the sensing circuits 244 and 246 and/or the data acquisition system 252 to determine or detect whether and to what degree tissue capture has occurred and to program a pulse, or pulses, in response to such determinations. The sensing circuits 244 and 246, in turn, receive control signals over signal lines 248 and 250 from the microcontroller 220 for purposes of controlling the gain, threshold, polarization charge removal circuitry (not shown), and the timing of any blocking circuitry (not shown) coupled to the inputs of the sensing circuits, 244 and 246, as is known in the art.

For arrhythmia detection, the device 100 utilizes the atrial and ventricular sensing circuits, 244 and 246, to sense cardiac signals to determine whether a rhythm is physiologic or pathologic. In reference to arrhythmias, as used herein, "sensing" is reserved for the noting of an electrical signal or obtaining data (information), and "detection" is the processing (analysis) of these sensed signals and noting the presence of an arrhythmia. The timing intervals between sensed events (e.g., P-waves, R-waves, and depolarization signals associated with fibrillation which are sometimes referred to as "F-waves" or "Fib-waves") are then classified by the arrhythmia detector 234 of the microcontroller 220 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy").

Cardiac signals are also applied to inputs of an analog-to-digital (A/D) data acquisition system 252. The data acquisition system 252 is configured (e.g., via signal line 251) to acquire intracardiac electrogram ("IEGM") signals, convert the raw analog data into a digital signal, and can store the digital signals for later processing and/or telemetric transmission to an external device 254. The data acquisition system 252 can be coupled to the right atrial lead 104, the coronary sinus lead 106, and the right ventricular lead 108 through the switch 226 to sample cardiac signals across any pair of desired electrodes.

The microcontroller 220 is further coupled to a memory 260 by a suitable data/address bus 262, wherein the programmable operating parameters used by the microcontroller 220 are stored and modified, as required, in order to customize the operation of the implantable device 100 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 102 within each respective tier of therapy. One feature of the described embodiments is the ability to sense and store a relatively large amount of data (e.g., from the data acquisition system 252), which data may then be used for subsequent analysis to guide the programming of the device. The memory 260 can also store the pressure data related to embodiments of the present invention.

Advantageously, the operating parameters of the implantable device 100 may be non-invasively programmed into the memory 260 through a telemetry circuit 264 in telemetric communication via communication link 266 with the external device 254, such as a programmer, transtelephonic transceiver, or a diagnostic system analyzer. The microcontroller 220 activates the telemetry circuit 264 with a control signal 268. The telemetry circuit 264 advantageously allows intracardiac electrograms and status information relating to the operation of the device 100 (as contained in the microcontroller 220 or memory 260) to be sent to the external device 254 through an established communication link 266.

The implantable device 100 can further include a physiologic sensor 270, commonly referred to as a "rate-responsive" sensor because it is typically used to adjust pacing stimulation rate according to the exercise state of the patient. However, the physiological sensor 270 may further be used to detect changes in cardiac output, changes in the physiological condition of the heart, or diurnal changes in activity (e.g., detecting sleep and wake states). Accordingly, the microcontroller 220 responds by adjusting the various pacing parameters (such as rate, AV Delay, V-V Delay, etc.) at which the atrial and ventricular pulse generators, 222 and 224, generate stimulation pulses. While shown as being included within the implantable device 100, it is to be understood that the physiologic sensor 270 may also be external to the implantable device 100, yet still be implanted within or carried by the patient. Examples of physiologic sensors that may be implemented in device 100 include known sensors that, for example, sense respiration rate, pH of blood, ventricular gradient, oxygen saturation, blood pressure and so forth. Another sensor that may be used is one that detects activity variance, wherein an activity sensor is monitored diurnally to detect the low variance in the measurement corresponding to the sleep state. For a more detailed description of an activity variance sensor, the reader is directed to U.S. Patent No. 5,476,483 (Bornzin et al.).

The implantable device 100 additionally includes a battery 276 that provides operating power to all of the circuits shown in FIG. 2. For a device that employs shocking therapy, the battery 276 should be capable of operating at low current drains for long periods of time (e.g., preferably less than 10 µA), and be capable of providing high-current pulses (for capacitor charging) when the patient requires a shock pulse (e.g., preferably, in excess of 2 A, at voltages above 200 V, for periods of 10 seconds or more). The battery 276 also desirably has a predictable discharge characteristic so that elective replacement time can be detected.

The implantable device 100 can further include magnet detection circuitry (not shown), coupled to the microcontroller 220, to detect when a magnet is placed over the implantable device 100. A magnet may be used by a clinician to perform various test functions of the implantable device 100 and/or to signal the microcontroller 220 that the external programmer 254 is in place to receive or transmit data to the microcontroller 220 through the telemetry circuits 264.

The exemplary implantable device 100 is also shown as including an impedance measuring circuit 278 that is enabled by the microcontroller 220 via a control signal 280. The known uses for an impedance measuring circuit 278 include, but are not limited to, lead impedance surveillance during the acute and chronic phases for proper performance, lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 278 is advantageously coupled to the switch 226 so that any desired electrode may be used.

In the case where the implantable device 100 is intended to operate as an implantable cardioverter/defibrillator device, it detects the occurrence of an arrhythmia, and automatically applies an appropriate therapy to the heart aimed at terminating the detected arrhythmia. To this end, the microcontroller 220 further controls a shocking circuit 282 by way of a control signal 284. The shocking circuit 282 generates shocking pulses of low (e.g., up to 0.5 J to 2.0 J), moderate (e.g., 2.5 J to 10 J), or high energy (e.g., 11 J to 40 J), as controlled by the microcontroller 220. Such shocking pulses are applied to the patient's heart 102 through at least two shocking electrodes, and as shown in this embodiment, selected from the left atrial coil electrode 126, the RV coil electrode 132, and/or the SVC coil electrode 134. As noted above, the housing 200 may act as an active electrode in combination with the RV electrode 132, and/or as part of a split electrical vector using the SVC coil electrode 134 or the left atrial coil electrode 126 (i.e., using the RV electrode as a common electrode).

Cardioversion level shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5 J to 40 J), delivered asynchronously (since R-waves may be too disorganized), and pertaining exclusively to the treatment of fibrillation. Accordingly, the microcontroller 220 is capable of controlling the synchronous or asynchronous delivery of the shocking pulses.

Now that exemplary details of the implantable device 100 have been provided, additional details of the various embodiments of the present invention will be provided.

A typical pressure sensor generates electrical signals indicative of changes in a sensed pressure. Thus, one or more wires may be used to connect a sensor to the device 100, as was described above. FIG. 2 illustrates an embodiment where two pressure signals P1 and P2 are coupled to the device 100 via terminals 211 and 213, respectively. An analog-to-digital (A/D) data acquisition system 253 may be configured (e.g., via signal line 255) to acquire and amplify the signals P1 and P2, convert the raw analog data into a digital signal, filter the signals and store the digital signals (e.g., in memory 260) for later processing by, for example, a pressure measurement processing component 238 and/or telemetric transmission to an external device 254. As mentioned above, it is also possible that the pressure sensors can produce digital signals. In such a case, the A/D 253 would not be needed. In addition to (or instead of) storing the pressure signals, it is also possible that the pressure signals be processing in real or near real time.

The implanted device 100 is also shown as including a patient alert 221, which can inform the patient that medical attention should be sought. In accordance with an embodiment, the alert 221 is provided through an electromechanical transducer that generates sound and/or mechanical vibration, that can be heard and/or felt by the patient. These are just a few examples of patient alerts, which are not meant to be limiting. One of ordinary skill in the art will appreciate that other types of patient alerts can be used, while still being within the spirit and scope of the present invention.

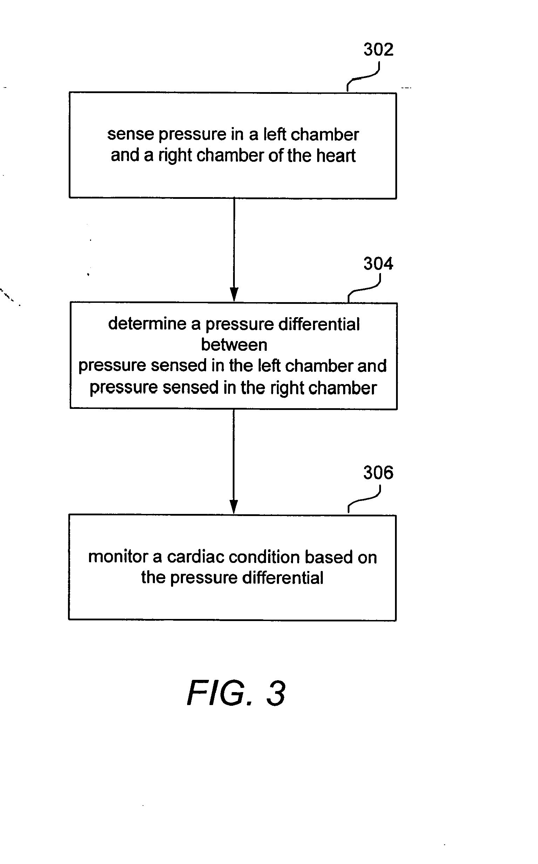

Additional details of specific embodiments of the present invention will now be described with reference to the high level flow diagram of FIG. 3. More specifically, the flow diagram of FIG. 3 will be used to describe how pressure differential measurements can be used to monitor one or more cardiac condition, in according with embodiments of the present invention.

Referring to FIG. 3, at a step 302 pressure is sensed in a patient's left atrium and in a patient's right atrium and/or ventricle. For example, referring back to FIG. 1B, the left atrial pressure sensor 145 can be used to sense pressure and the left atrium, and the right atrial pressure sensor 147 can be used to sense pressure in the right atrium. If pressure is sensed in the right ventricle instead of (or in addition to) the right atrium, then the right ventricular sensor 165 can be used to sense right ventricular pressure.

At a step 304 a pressure differential is determined between the sensed pressure in the left atrium and the sensed pressure in the right atrium or ventricle. For example, a left atrial pressure signal (or measurements thereof) can be subtracted from the right atrial pressure signal (or measurements thereof), or vice versa, to produce a pressure differential. Alternatively, the left atrial pressure signal (or measurements thereof) can be subtracted from the right ventricle pressure signal (or measurements thereof), or vice versa, to produce the pressure differential. By determining pressure differentials, as opposed to absolute pressures, calibrations/adjustments for changes in weather, altitude or similar pressure affecting factors are not necessary, since the pressure in the left and right chambers should both be equally affected by such changes. Accordingly, with embodiments of the present invention, an external (i.e., non-implanted) pressure sensor is not needed for measuring ambient pressure.

With embodiments of the present invention, if the pressure differential stays relatively constant, while the pressure in the left atrium and the pressure in the right atrium or ventricle both change by similar magnitudes, it can be assumed that the changes in pressure are due to non cardiac environmental conditions, such as changes in weather and/or altitude.

In some embodiments of the present invention, if the pressure differential changes, there is also a determination of whether the change in the pressure differential can be attributed to a pressure change in only one of the chambers, while pressure in the other chamber stays relatively constant. For example, if there is a rapid increase in the pressure differential, while at the same time there is a rapid increase in the left atrial pressure that is of a similar magnitude to the increase in the differential pressure, but not a rapid increase in the right atrial pressure, then it can be assumed that the rapid increase in the differential pressure is due primarily to a rapid increase in the left atrial pressure while the right atrium pressure remains relatively constant. This is just one example of how this can be accomplished. Similarly, an increase in right atrial pressure not accompanied by a concomitant increase in left atrial pressure suggests a progression of isolated right heart failure or the development of pulmonary hypertension. One of ordinary skill in the art would appreciate that other techniques are also within the spirit and scope of the present invention. As will be explained below, this information can be used to discriminate between certain cardiac conditions, e.g., between pulmonary hypertension and pulmonary edema.

At a step 306, a cardiac condition is monitored based on the pressure differential. Examples of the cardiac conditions that can be monitored include left-sided heart failure, right-sided heart failure, biventricular failure, pulmonary edema and pulmonary hypertension, each of which is briefly discussed below. The pressure differential data and/or pressure data from individual chambers can be stored in the memory 260, enabling the device 100 (and more specifically, e.g., the pressure measurement processing module 238) to track trends in the pressure differential.

Embodiments of the present invention can be used to detect pulmonary hypertension and other pulmonary problems. Unlike a single pressure sensor located in a single (e.g., right) chamber of the heart, embodiments of the present invention can be used to readily distinguish pulmonary hypertension from left-sided heart failure and other cardiac conditions. Embodiments of the present invention can also be used to detect both left sided and right sided heart failure disease progression, as will be described below.

Left-sided heart failure occurs when the heart is not able to meet the perfusion requirements of the body. The body attempts to improve cardiac output by retaining fluid, i.e., it responds in a way that would be appropriate if the reduce perfusion were due to blood loss. The excess fluid increases the filling pressure of the left heart, and often also escapes into the pulmonary tissues, resulting in difficulty in breathing. Using the present invention, an increase in the pressure differential can be recognized as a worsening of left-sided heart failure. The pressure differential will increase because the left atrial pressure sensor will measure the increase in the left ventricular filling pressure. This pressure increase can develop over minutes to weeks, depending on the underlying pathophysiology. For example, if the patient has a myocardial infarction which involves a papillary muscle supporting the mitral valve, he or she can develop sudden onset, severe mitral regurgitation and flash pulmonary edema from the increased pulmonary venous pressures. This can occur in minutes. At the other extreme is a patient with relatively stable but worsening heart failure, in which the left-sided filling pressures gradually increase over weeks to months. Accordingly, in accordance with an embodiment of the present invention, if there is an increase in the pressure differential by more than a specified threshold, then such an increase can be recognized as a worsening of left-sided heart failure. Such a threshold will likely be selected based on empirical data. Multiple thresholds can be used to distinguish between the different potential causes for left-sided heart failure. For example, an increase in the pressure differential beyond a specified amount over less than a specified time period (e.g., 1 hour), can be recognized as a sudden onset of severe mitral regurgitation and/or flash pulmonary edema. For another example, an increase in the pressure differential beyond a specified amount over more than a specified time period (e.g., 10 days), can be recognized as a gradual worsening of left-sided heart failure.

Right-sided heart failure heart failure, which is typically caused by damage to the right sided chambers of the heart, leads to decreased blood flow, and swelling hands, lungs and abdomen. Using the present invention, a gradual decrease in the pressure differential can be recognized as a gradual worsening of right-sided heart failure. In a similar manner as was discussed above, multiple thresholds can be used to distinguish between different underlying pathophysiologies.

Pulmonary edema is a condition where fluid builds up on the lungs, thereby blocking transportation of oxygen from the lungs into the blood. Using the present invention, a rapid increase in the pressure differential, while pressure in the right atrium or ventricle remain relatively constant, is recognized as pulmonary edema. More specifically, such a rapid increase in the pressure differential would be indicative of a rise in the hydrostatic pressure in the pulmonary vein back into the lungs, which would cause an imbalance between the hydrostatic forces and the oncotic forces that would increase the level of interstitial fluid in the pulmonary region. The term rapid as used herein refers to a time frame of a few minutes to a few days (e.g., two days). Accordingly, in accordance with an embodiment of the present invention, if there is an increase in the pressure differential by more than a specified threshold over a time frame of a few minutes to a few days, while the pressure in the right chambers of the heart remain relatively constant, then such an increase can be recognized as an exacerbation of pulmonary edema.

A rapid increase in the pressure differential may be due to a number of reasons such as flow restrictions in the pulmonary artery manifesting itself as pulmonary hypertension. Pulmonary hypertension is a condition that occurs when blood pressure in the blood vessels supplying the lungs is too high. This increased pressure causes the right ventricle to become enlarged, and may result in fainting, chest pain and heart failure. Using the present invention, a rapid increase in the pressure differential, while the sensed pressure in the left atrium remains relatively constant, is recognized as pulmonary hypertension.

Endocardial flow, which is the flow of blood through the heart, is a surrogate for cardiac output. Using the present invention, pressure differential measurements can be used to monitor endocardial flow, and thus cardiac output. In such embodiments, if the pulmonary vascular resistance remains constant or approximately constant, increases in the pressure differential are indicative of increased endocardial flow (and thus increased cardiac output), and decreases in the pressure differential are indicative of decreases in endocardial flow (and thus decreases in cardiac output). Since reductions in cardiac output are believed to be indicative of worsening heart failure, monitoring endocardial flow based on changes in pressure differential can be used to monitor progression of heart failure.

One or more response can be triggered if an exacerbation of a cardiac condition, such as pulomonary edema or pulmonary hypertension, is detected. For example, the patient alert 221 can be triggered when an exacerbation is detected. As was described above, the patient alert 221 can produce an audible or vibrational warning signal indicating that the patient should consult with a physician.

Preferably, the implanted device can analyze the pressure data to detect such exacerbations, e.g., using the pressure measurement processing module 238 of the microcontroller 220. That is, in accordance with specific embodiments of the present invention, the implanted device 100 automatically analyzes the data it acquires, recognizes a worsening of disease status, and notifies the patient when appropriate of the need for physician consultation.

In certain embodiments, an external telemetry unit can be available in the patient's home or some other frequented location. When the implanted monitor recognizes worsening disease status, the patient is notified, and/or data is telemetered to the external telemetry unit and via the telephone or other communication lines to a physician and/or to a central location for further review.

There are a variety of levels of data processing that can be performed by the implanted device 100. For example, in certain embodiments the implanted device 100 may simply store raw data for later retrieval and analysis by a physician or clinician. In this embodiment, the device 100 functions primarily as a tool that allows the physician to optimize medical therapy or work up a possible exacerbation. Alternatively, the raw data might be stored over an extended but relatively short period of time, such as 24 hours, and periodically and routinely conveyed by means of the transmitter to an external module which performs high level diagnostic analysis or data archiving.

In yet another embodiment data is routinely and automatically conveyed to the external telemetry unit which performs more computationally intensive analysis or delivers the data via communication lines to a central location for further analysis or review.

In a preferred embodiment, the microcontroller 220 derives a high-level clinical diagnosis from the collection of pressure sensor outputs. For example, the pressure measurement processing module 238 of the micro-controller 220 might deduce that an acute heart failure exacerbation is developing and that the patient and physician should be notified. In this case the device 100 would activate the patient alert 221 to inform the patient that medical attention should be sought. A physician can then review the data, interview the patient, and determine what course of action is appropriate.

As explained in detail above, by using pressure differential measurements, an external pressure sensor measuring ambient pressure as a reference and a source of calibration is not required. The pressure differential or "Delta," which is equal to the difference between the left atrial pressure and the right atrial or ventricular pressure, can be tracked by the implanted system and short term and long term changes may indicate significant changes in the patient heart disease condition.

While embodiments of the present invention monitor cardiac conditions based on changes in pressure differential, this disclosure does not limit other measurements that could be made from the left atrial pressure signal and the right atrium or ventricle pressure signals, including dP/dT, etc.

## Claims

1. An implantable system for monitoring a cardiac condition, **characterised by**:
means for determining a pressure differential (145,147) between pressure sensed in a left chamber and pressure sensed in a right chamber of the heart (102); and
means for monitoring (220) a cardiac condition based on the pressure differential.

2. A system as claimed in Claim 1, **characterised by**: a first pressure sensor (145) to be implanted in the left atrium or ventricle to sense pressure in a left chamber of the heart (102); and a second pressure sensor (147) to be implanted in the right atrium or ventricle to sense pressure in a right chamber of the heart (102).

3. A system as claimed in Claim 1 or Claim 2, **characterised in that** the means for monitoring (220) recognizes an increase in the pressure differential as a worsening of left-sided heart failure.

4. A system as claimed in Claim 3, **characterised in that** the means for monitoring (220) detects a gradual increase in the pressure differential when the pressure differential increases by at least a specified threshold amount over a specified threshold time period or more.

5. A system as claimed in Claim 1 or Claim 2, **characterised in that** the means for monitoring (220) recognizes a rapid increase in the pressure differential, while pressure sensed in the right chamber remains relatively constant, as pulmonary edema.

6. A system as claimed in Claim 1 or Claim 2, **characterised in that** the means for monitoring (220) recognizes a rapid increase in the pressure differential, while pressure sensed in the left chamber remains relatively constant, as pulmonary hypertension.

7. A system as claimed in Claim 5 or Claim 6, **characterised in that** the means for monitoring (220) detects a rapid increase in the pressure differential when the pressure differential increases by at least a specified threshold amount over a specified threshold time period or less.

8. A system as claimed in Claim 1 or Claim 2, **characterised in that** the means for monitoring (220) recognizes a gradual decrease in the pressure differential as a worsening of right-sided heart failure.

9. A system as claimed in Claim 8, **characterised in that** the means for monitoring (220) detects a gradual decrease in the pressure differential when the pressure differential decreases by at least a specified threshold amount over a specified threshold time period or more.

10. A system as claimed in any preceding Claim, **characterised in that** the means for monitoring (220) monitors endocardial flow.

11. An implantable system for monitoring a cardiac condition as claimed in Claim 1, **characterised by**: a first pressure sensor (145) to be implanted in the left atrium or ventricle to sense pressure in a left chamber of the heart (102); a second pressure sensor (147) to be implanted in the right atrium or ventricle to sense pressure in a right chamber of the heart (102); and a microcontroller (220) to determine a pressure differential between pressure sensed by the first pressure sensor (145) and pressure sensed by the second pressure sensor (147) and to monitor a cardiac condition based on the pressure differential.
